# EUROPEAN PATENT APPLICATION

(11) **EP 2 387 940 A1**
(43) Date of publication of application: **23.11.2011**
(21) Application number: 10450165.5
(22) Date of filing: 28.10.2010
(51) Int. Cl.: A61B 5/00, G01N 33/00, G01N 33/574, A61B 6/02, G03B 42/08, G01N 21/62, A61B 6/00, G01T 1/10

(54) **Luminescence probing in strongly scattering objects using ionizing radation**

(71) Applicant: Technische Universität Graz, 8010 Graz (AT); Forschungsholding TU Graz GmbH, 8010 Graz (AT)
(72) Inventor: Scharfetter, Hermann, 8045 Graz (AT); Freiberger, Manuel, 8010 Graz (AT); Gürsoy, Doga, 8010 Graz (AT)
(74) Representative: Patentanwaltskanzlei Matschnig & Forsthuber OG

(57) **Abstract**

The invention relates to a method for diffuse luminescence probing in strongly scattering objects (1), comprising the following steps: a) Bringing a luminophore substance into the object (1), the luminophore substance emitting light that is detectable by photodetectors (8) when irradiated with ionizing radiation (4); b) Irradiating the object (1) with ionizing radiation (4); c) Detecting the resulting luminescence light with at least one photodetector (8); d) Analyzing the detected information. The invention further pertains to an apparatus for performing said method.

## Description

### Field of the invention and description of prior art

The invention relates to a method for diffuse luminescence probing in strongly scattering objects. The invention further relates to an apparatus for performing aforementioned method.

Luminescence is optical radiation (i.e., different forms of light) that is emitted by an object at low temperatures (e. g. room temperature). Photoluminescence is luminescence which has been excited by incident radiation, e.g., laser light. Photoluminescence is further classified in fluorescence and phosphorescence which are typically associated with different decay times (i.e., the duration of light emission or glowing after the excitation). A very short persistence in the range of nanoseconds is referred to as "fluorescence" while a glowing of up to several milliseconds is called "phosphorescence". Radioluminescence is luminescence stimulated by ionizing radiation, e.g., Gamma-radiation.

In the following disclosure, all mentioning of luminescence pertains to photoluminescence and in particular to fluorescence, phosphorescence and radioluminescence. This also applies to substances that show abovementioned behavior: The term "luminophor" refers to substances which emit fluorescence or phosphorescence light when irradiated with subatomic particles (Gamma-radiation) or electromagnetic radiation of shorter wavelength in the spectrum from X-ray radiation to near infrared (NIR) light.

Luminescence is widely applied in different fields. In particular, fluorescence techniques are used in biomedical sciences, for making visible processes at the molecular level. Examples are fluorescence microscopy, 2D in-vivo fluorescence imaging and, most recently, fluorescence diffuse optical tomography (FDOT). All these technologies employ the ability of fluorescent dyes to absorb light in a certain wavelength range and to re-emit photons at a larger wavelength.

In a typical experiment, the investigated sample, which can be a slice of tissue or a small animal, is excited through a set of light sources placed on the surface of the sample. The excitation light then propagates in a diffuse manner through the tissue and is partially absorbed by fluorophores, which re-emit part of the light at a longer wavelength. The emitted light then propagates back through the tissue, again in a diffuse manner, and is recorded by detectors at the boundary. A particularly advantageous feature of fluorescence imaging is that the activity of fluorophores is influenced by metabolic states or processes and thus enables functional imaging, e.g. to determine physiological activities in biological systems.

FDOT is one of the leading emerging technologies for molecular imaging in small animals. It is described in CN 101 301 192 A, for instance. It enables the mapping of molecular processes in strongly scattering media, especially in biological tissues. However, despite its desirable properties, FDOT suffers from one major disadvantage: The diffuse light propagation inside of the highly scattering tissues leads to low image resolution especially towards the center of the target. Therefore, fluorophore concentrations in the central regions of a specimen are very difficult to resolve and quantification is inaccurate.

Nowadays, in order to achieve good FDOT results one needs to build accurate reconstruction models which employ the exact anatomy of the object or animal as a-priori information. This information is usually obtained from X-ray computed tomography (CT) or magnetic resonance imaging. However, small modeling errors or inaccurate registration can lead to significant errors and artifacts; furthermore, the scattering problems of the incident light remain.

The propagation of light in highly scattering media is governed by a diffusion equation (photons are scattered many times while passing through the tissue), which not only limits the spatial resolution of fluorescence optical tomography, but also renders the reconstruction an ill-posed inverse problem. In order to achieve a stable inversion, one has to use regularization methods and utilize all available a-priori information in the formulation of the inverse problem. This information can be a-priori knowledge about the distribution of the fluorescent dye or estimates for the covariance of the measurement data. Irrespective of the used regularization method image resolution decreases dramatically with the distance from the surface. Therefore, fluorophore accumulations in the central regions of an object, e.g. a mouse or another small animal, are very difficult to resolve and the quantitative estimation of the fluorophore concentration is inaccurate.

This is because the diffuse scattering process occurs twice: First, the incident light is scattered when traveling from the surface to the deeper regions of the object and second the emission light propagates in a diffuse manner from the excited fluorophore towards the surface. For highly scattering media such as most biological tissues the light propagation inside a domain is described by two coupled partial differential equations. The image reconstruction is achieved by the inversion of a discretized form of said equations in order to obtain the fluorophore concentration and, in the case of complex data, also the fluoresecence lifetime. However, the nature of the mathematical equations which describe the scattering of the incident and emergent light limits the spatial resolution in a fundamental manner.

### Summary of the invention

It is therefore a goal of the present invention to provide a method to ameliorate the quality of images acquired by luminescence probing.

This goal is met by a method as stated in the beginning, comprising the following steps:
a) Bringing a luminophore substance into the object, the luminophore substance emitting light that is detectable by photodetectors when irradiated with ionizing radiation;
b) Irradiating the object with ionizing radiation;
c) Detecting the resulting luminescence light with at least one photodetector;
d) Analyzing the detected information.

The basic concept of this invention is to excite the luminophore substance by ionizing radiation rather than light, thereby obviating the first diffuse pathway, i.e. the diffusion of the excitation light. The ionizing radiation might be X-ray radiation or gamma radiation, to name only two possible candidates; generally speaking, all subatomic particles and electromagnetic waves energetic enough to detach electrons from atoms or molecules are subsumed under the term "ionizing radiation" and may be used here. X-ray radiation in the present disclosure denotes electromagnetic radiation with a wavelength in the range of 0,01 nm to 10 nm, typically produced by an X-ray tube or other appropriate sources. Gamma radiation is electromagnetic radiation with very short wavelength and high frequency emanating from the atomic nucleus or from other particle decays or annihilation events.

Ionizing radiation is scattered much less in optically strongly scattering objects like biological tissues than light and can travel in nearly straight beams - the excitation pathways are well-defined rather than diffused. Hence, the invention seeks to improve the lack of resolution by obviating the diffuse excitation pathway. Analyzing the information of the photodetector in step d) allows to reconstruct the internal distribution of the luminophore substance in the object. For the analysis in step d) procedures may be used that are already well-established in X-ray CT, for instance, and well known to any person skilled in the art. Hence, no further elaboration about how to analyze the detected information is included here.

The term "photodetector" here denotes sensors that are capable of detecting light in a wide range of wavelengths, typically from 100 nm to 1000 nm. It includes optical detectors, photodiodes, photomultiplier tubes, charge-coupled devices (CCD) and the like.

In contrast to conventional X-ray imaging and CT the method provides information about physiological states and processes and is sensitive to changes in the optical properties (e.g., life-time or conversion efficiency) of a suitable luminophore substance.

Because the luminophores emit light in a wavelength-range that is detectable by photodetectors in a variant of the invention a luminophore substance is chosen (step a)) that emits light in the range of 600 nm to 900 nm when irradiated with X-ray radiation - hence, detectors that are already in use for conventional FDOT may be used in the method. By choosing this wavelength range the luminescence light can penetrate the material surrounding the luminophore substance, e.g., biological tissue mass. Light with another wavelength would be absorbed, hence decreasing signal quality. Therefore, abovementioned wavelength range is particularly advantageous. However, other wavelength ranges could be chosen as well, depending on the application of the method.

With luminophore substances which emit visible or near infrared (NIR) light when hit by ionizing radiation like X-rays the emission remains strictly localized along a beam. In a nutshell, by excitation of probing luminophore substances with ionizing radiation along well-defined pathways the invention provides a significant improvement of image resolution and better quantification, mitigating many imaging artifacts which are caused by the ill-posedness of the inverse FDOT-problem.

The range of possible applications is very large, covering also all applications of luminescence probing in highly scattering objects like biological tissues and animals, including in-vivo molecular fluorescence imaging, but with higher spatial resolution than conventional optical techniques.

In another (preferred) variant of the invention, in step a) the luminophore substance is a mixture comprising a first substance and at least one second substance wherein the first substance absorbs ionizing radiation and emits light in a wavelength range that excites the second substance (fluorophore or phosphore) so it emits light that is detectable by photodetectors and/or that is in the range of 600 nm to 900 nm. Advantageously, the mixture is nanostructured. This allows to combine scintillating agents that are excitable by ionizing radiation with luminophores which emit light that is detectable by photodetectors. To this end appropriate luminophores are linked to such scintillating agents. To have efficient energy transfer, the emission peaks of the scintillating agents must effectively overlap with the absorption or excitation peaks of the luminophores. This variant of the invention enables the use of specific luminophores as second substance, namely Pt- or Ir-complexes which are established for chemical sensing (e. g. for oxygen).

Advantageously the luminophore substance comprises scintillation particles like LaF₃:Tb³⁺ nanoparticles and/or LaF₃:Ce³⁺, Tb³⁺ nanoparticles. Scintillation here denotes the property of emitting light, typically in the visible range, when excited by ionizing radiation. Abovementioned substances are known from photo-chemotherapy where they are combined with photosensitizers; the nanoparticles absorb X-ray radiation and emit light with high quantum yield so the photosensitizers generate singlet oxygen for deep cancer treatment. The singlet oxygen augments the killing of cancer cells by ionizing radiation. This is described by Chen and Zhang in "Using Nanoparticles to Enable Simultaneous Radiation and Photodynamic Therapies for Cancer Treatment" (J. Nanosci. Nanotechnol., Vol. 6, 1159-1166, 2006). Other doped nanoparticles that could be used here include LuF₃:Ce³⁺, CaF₂:Mn²⁺, CaF₂:Eu²⁺, BaFBr:Eu²⁺, BaFBr:Mn²⁺ and CaPO₄:Mn²⁺, or semiconductor nanoparticles like ZnO, ZnS and TiO₂ to name only a few.

All particles used in the method need to fulfill certain preconditions besides the ability to be excited by ionizing radiation, e.g., not being toxic, possibility to link them to luminophores and high quantum efficiency. A combination of an appropriate luminophore with abovementioned scintillating particles results in a mixture where the luminophore transforms the primary emission light into a secondary emission light at higher wavelengths (ideally in the near infrared band); such a mixture is well suited for the method according to the invention.

The method according to the invention may be performed with a pencil-beam of ionizing radiation scanning across the sample. In a variant of the invention in step b) the ionizing radiation has a fan-like shape. The fan-beam system uses a point source of radiation that emanates a fan-shaped beam and allows for irradiation of a larger area of the examined object. Preferably, more detectors for measuring the resulting fluorescent light are provided to account for the broad excitation of the sample. This means a significant improvement of the ill-posedness off the optical reconstruction problem of conventional methods since the reconstruction then relies on the multiple excitations with various beams of ionizing radiation from different directions.

The ionizing radiation may be irradiated in different modes in step b), namely in short pulses and/or by modulating the radiation source that emits the ionizing radiation with a certain frequency. Thereby even luminescence lifetime imaging can be achieved. In case of compound luminophore substances comprising a scintillator the scintillator must fulfill an additional requirement: Its scintillation decay time must be shorter or at least not noticeably longer than the luminescence lifetime of the linked fluorophore or phosphore. The modulation frequency may be 100 MHz and above for the measurement of fluorescence lifetime and several kHz to some hundreds of kHz for typical phosphorescence lifetimes.

Synchronous demodulation may take place by using a gated image intensifier so as to extract the phase information which is necessary to recover luminescence lifetime.

In another embodiment of the invention steps b) and c) are repeated and for each iteration the incidence angle of the ionizing radiation to the object is changed by moving the source of the ionizing radiation along an orbital path around the object. The number of repetitions depends on how much of the object should be scanned and in which increments the incidence angle is changed - obviously, a complete turn around the objects measures 360°, hence an increment of 20° from one iteration to the next results in 18 repetitions altogether.

The term "orbital" includes circular as well as elliptic paths, depending on the desired implementation of the method and the mechanical circumstances. The path may be positioned in a plane parallel or perpendicular to a longitudinal axis (i.e., the axis of rotation of the source) of the examined object. In the case where the plane is parallel to the axis of rotation the special case where the axis of rotation lies in said plane is included. If the object is positioned on a bearing surface the path may run in parallel or perpendicularly to said surface.

The - more or less - orbital movement around the object (or an axis of rotation inside the object) may be combined with a movement along the object, i.e. parallel to the axis of rotation, resulting in an overall spiraled path of the source around and along the object.

The results of the method according to the invention can be further improved when in step b) the object is additionally irradiated by a non-ionizing light source. This allows a combination of "conventional" optical excitation with luminescence excited by ionizing radiation and yields additional information about the examined subject. By and large, a light source is used that emits light in the visible range with wavelengths from 600 nm to 800 nm, but also lasers with wavelengths ranging from 100 nm (far ultraviolet) 1500 nm (far infrared) may be used - again, the light source most suited for the job depends on the luminophore substance used.

In a variant of the invention for the detection in step c) a plurality of photodetectors is arranged on each side of the object. In step c) this allows for detecting the entire emitted light power and in step d) the detected light can be analyzed by integrating over a closed surface around the target object. This variant of the invention is particularly well suited for objects that are only weakly absorbing and mainly scatter the luminescence light. The integral of the closed surface then corresponds to the absorption line integral measured in X-ray CT and the optical reconstruction problem of step d) gets very similar to the standard reconstruction problem of CT; hence, well established methods can be used as a staring point for a proper analysis of the measured data of the detectors.

In yet another embodiment of the invention X-ray radiation is used in step b) and in step c) also the X-rayradiation permeating the object is detected by at least one X-ray detector. The detectors are positioned in the vicinity of the examined object. This essentially allows performing conventional X-ray CT by measuring the transmitted (and, reversely, the absorbed) radiation. The information of both detectors, the photodectectors and the X-ray detectors, is used in step d).

When combining the method according to the invention with X-ray CT the X-ray absorption along any path can be calculated from the CT images and corrected for in the optical reconstruction. Additionally, a combination with X-ray CT yields morphological information which can be directly used for regularizing the inverse image reconstruction problem. Both effects reduce the ill-posedness of the latter significantly and hence yield a much more accurate imaging.

This variant of the method according to the invention allows a realization in already existing micro-CT facilities by furnishing them with photodetectors (and the appropriate processing means): If the effort of X-ray-CT is made anyway (e.g. for FDOT with overlayed CT images) the same X-rays can be used for luminescence excitation. Therefore image registration becomes much easier and less prone to artifacts as the sources for both imaging modalities are the same - there are less possibilities for systematic errors..

The invention furthermore pertains to an apparatus for diffuse luminescence probing in strongly scattering objects according to abovementioned method, the apparatus comprising at least one source of ionizing radiation and at least one photodetector and a processing unit for analyzing the data from the detector. The photodetector may be sensitive to light with wavelengths in the range of 600 nm to 900 nm. The processing unit may be a computer, for instance.

In a variant of the invention the apparatus further comprises at least one detector for ionizing radiation. If X-ray radiation is used and the detector is suitable for detecting X-ray radiation this enables performing a combination of the method according to the invention and conventional X-ray CT. In another variant of the invention the apparatus further comprises at least one non-ionizing light source. The light source may be a laser, for instance. In yet another variant of the invention the source of ionizing radiation may be movable along an orbital path around the object.

### Brief description of the drawings

In the following, the present invention is described in more detail with reference to the figures, which schematically show:
Fig. 1 a schematical plan view of an apparatus for performing the method according to the invention, and
Fig. 2A-C different variants of performing the method according to the invention.

### Detailed description of the invention

The method according to the invention is now explained by means of one possible embodiment. It should be stressed here that the invention is not restricted to the procedures and applications presented in the following which merely serve to illustrate the method.

The method shows a combination of X-ray induced luminescence and conventional X-ray CT - however, it should be noted that the inclusion of X-ray CT is an optional and not a compulsory step of the method according to the invention. Furthermore it is noted that the term "Luminescence" in the following pertains to photoluminescence and in particular to fluorescence, phosphorescence and radioluminescence, or to photoluminescence which is stimulated by radioluminescence.

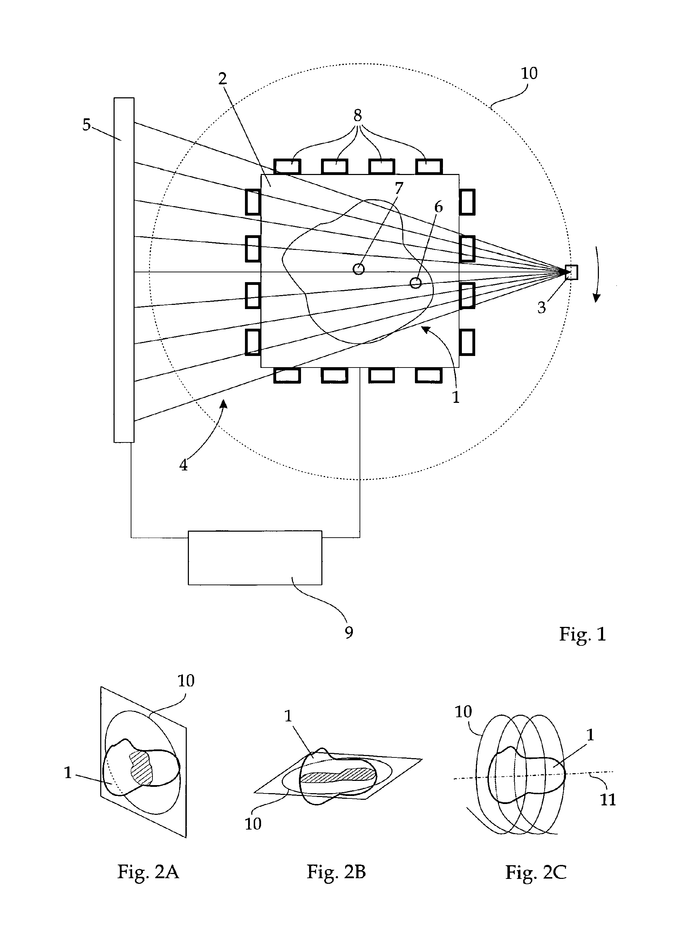

In Fig. 1 the examined object 1 is positioned on a bearing surface 2. The object 1 could be a slice of tissue or a small animal, for instance. The X-ray source 3 for irradiating the object 1 with X-ray radiation is modeled as a fan beam system which uses a point source of radiation that emanates a fan-shaped beam of rays 4. For the sake of visibility the bundle of rays 4 only shows nine rays where in reality a - more or less - homogeneous fan would irradiate the object 1. The rays run from the X-ray source 3 across the bearing surface 2 towards an X-ray detector screen 5. The X-ray detector screen 5 consists of a number of detectors for measuring the X-ray intensities for the sake of conventional X-ray CT. As mentioned above this is only an optional step of the method according to the invention.

However, when the method is implemented in existing facilities (e.g., by equipping them with appropriate photodetectors) this is a natural setup. If the additional effort of X-ray CT is made anyway (which is frequently done), the same X-ray radiation can be used for luminescence excitation. Then the image reconstruction (step d)) becomes much easier because the excitation pathways are well-defined and the morphological information can be used for significantly better conditioning the diffuse optical subproblem. The resulting images are much less prone to artifacts so that such a X-ray/optical combo-system would be revolutionary for small animal imaging.

The angle between the X-rays is assumed to be constant. The view-angle of the X-ray source 3 is taken as 45 degrees, for instance.

In step a) of the method according to the invention luminophore substances are brought into the object 1 to be examined. The method uses luminophore substances that emit light in the visible to NIR range (i.e., in a wavelength range that is detectable by photodetectors and easily propagates through the tissue) when irradiated with X-ray radiation. The luminophore substance could also be a mixture of scintillating agents which transform X-ray radiation (or Gamma-rays) into visible light and luminophores that absorb this light and emit visible or NIR light that is detectable by photodetectors and easily propagates through the tissue. To have efficient energy transfer, the emission peaks of the scintillating agents must effectively overlap with the absorption or excitation peaks of the luminophores.

The scintillating agents could be nanoparticles which have a number of advantages, e.g. that they can be made hydrophilic, possess relatively large surface areas for connecting with the luminophores and can penetrate deep into objects, particularly tissue.

In the example presented here after step a) the object to be examined contains two different fluorescent dye inclusions 6, 7 at different positions. The first inclusions 6 represents a local fluorescent dye near the surface of the object 1 whereas the second inclusions 7 is placed in a central (deep) position. The inclusions 6, 7 may result from smart probes, specifically targeting certain structures or receptors (e.g., in organs or tumors). Furthermore, non-specific luminophores may be used that disperse in an object according to their pharmacokinetics. Examples for such non-specific luminophores are Pt- or Ir-complexes (in combination with appropriate scintillating agents) for indirect chemical sensing (e.g., phosphorescence decay time).

Regardless of which luminophores are chosen for the method, preferably the light they emit after excitation lies in the range of 600 nm to 900 nm. Light with such wavelengths is least absorbed in objects the method according to the invention is most likely to be used with. Depending on the object, other wavelength ranges could be used as well; however, objects which contain water strongly absorb light with wavelengths above 900 nm.

In step b) the object 1 is irradiated by X-ray radiation emanating from an X-ray source 3. In other embodiments of the invention more than one source can be used to accelerate the process or increase accuracy of the method. It has to be noted that it is also possible to use other forms of ionizing radiation (and other radiation sources) like gamma rays, for instance.

The radiation can be irradiated in different ways, either with a continuous wave with constant intensity or modulated intensity (leading to frequency domain methods) or with short pulses (allowing time of flight measurements). The X-ray intensity may be modulated by using X-ray tubes with control grids which are then used to apply the high frequency control voltage. Also a mechanical shutter can be used which absorbs the X-ray radiation when closed. With such shutters modulation frequencies in the kHz-range appear feasible. Such a setup could be used for phosphorescence lifetime measurements.

The duration of the short pulses must be shorter than the expected luminescence lifetime, e.g. 100 ps for fluorescence lifetime or several microseconds for phosphorescence lifetime measurements. When using mixtures of scintillating agents and luminophores the pulse duration must stay reasonably below the scintillation lifetime.

In a variant of the invention (not pictured) the object 1 is additionally irradiated with a "conventional" light source, leading to conventional luminescence. This allows for combination of conventional luminescence probing methods with the method according to the invention. Such a light source could be a laser, for instance.

In step c) the resulting light from the object 1 is detected by appropriate photodetectors - in the variant described here, 16 photodetectors 8 are used, four on each side of the bearing surface 2. It is also possible to use more or less detectors, arranging them differently (e.g., directly on the object 1) and on additional sides of the object 1. Also a circular arrangement is possible so that all emitted light is detected. Furthermore, the X-ray radiation is measured by X-ray detectors 5 which measure the absorbed intensity and the like.

In the variant of the method described here, steps b) and c) are repeated several times. In each iteration the incidence angle of the X-ray radiation to the object 1 is changed by moving the X-ray source 3 along an orbital path 10 (the dotted circle in the figure) around the object 1. If necessary, the detectors may move synchronous to the source in order to secure proper measurements.

This is a case where the orbital path 10 lies in a plane which is parallel to the bearing surface 2 (Fig. 2A). The axis of rotation (not pictured) of the X-ray source 3 is perpendicular to the image plane in Fig. 1 and the bearing surface 2. In another variant (Fig. 2B) the orbital path may be located in a plane perpendicular to the bearing surface 2. In this case, the axis of rotation would (approximately) be located in the image plane of Fig. 1. In yet another variant which is pictured in Fig. 2C the X-ray source 3 may move along the axis of rotation 11 in addition to the circular movement, resulting in a spiralled path around and along the object. Such spiralling trajectories allow true 3-D-imaging.

The number of iterations depends on the step-size. With steps of 12 degrees 30 iterations are necessary for a complete tour around the object 1. In Fig. 1 the source moves in a plane that is parallel to the image plane (see also Fig. 2A). However, as described above the orbital path could also be perpendicular to the image plane or take any angle between parallel and perpendicular. Also, the path may not only be circular but also elliptical or any form.

In step d) the measurements of the detectors (the photodetectors 8 as well as the X-ray detectors 5) are analyzed. This is done with an appropriate processing unit 9 - a computer with sufficient computing power, for instance. It reconstructs the internal luminophore distribution and morphological as well as general anatomical information from the measurements of the detectors.

In a variant of the invention in step c) the whole emitted light power is detected (e.g. with photodetectors on every side of the object) and in step d) the detected light is then analyzed by integrating over a closed surface around the target object.

Since it is well known from luminescence diffuse optical tomography and conventional X-ray CT how to reconstruct images and information from measurement data the necessary algorithms and procedures are not discussed here.

From the above it follows that an apparatus for performing the method according to the invention should comprise at least one source of ionizing radiation, at least one photodetector and a processing unit 9 for analyzing the data from the detector. Preferably, the ionizing source 3 (and the detectors) is movable around the examined object 1 on an orbital or spiralled path, for instance.

As explained above, ionizing radiation denotes subatomic particles or electromagnetic waves energetic enough to detach electrons from atoms or molecules, thus ionizing them. However, even though ionizing radiation is used ionization does not necessarily have to happen: All kinds of excitation resulting in the emittance of visible light in the range of 600 nm to 900 nm are suitable for the method. Examples for appropriate ionizing radiation are X-ray radiation and gamma radiation.

Further providing an X-ray detector allows to include conventional X-ray CT to the method when X-ray radiation is chosen to excite the object to be examined. With at least one additional non-ionizing light source - e.g., a laser - "conventional" diffuse luminescence probing can be performed as well.

For human applications safety aspects have to be considered carefully for both the fluorescent dyes as well as for the X-ray application.

## Claims

1. Method for diffuse luminescence probing in strongly scattering objects (1), comprising the following steps:
a) Bringing a luminophore substance into the object (1), the luminophore substance emitting light that is detectable by photodetectors (8) when irradiated with ionizing radiation (4);
b) Irradiating the object (1) with ionizing radiation (4);
c) Detecting the resulting luminescence light with at least one photodetector (8);
d) Analyzing the detected information.

2. Method of claim 1, wherein in step a) a luminophore substance is chosen that emits light in the range of 600 nm to 900 nm when irradiated with X-ray radiation.

3. Method of claim 1, wherein in step a) the luminophore substance is a mixture comprising a first substance and at least one second substance wherein the first substance absorbs ionizing radiation (4) and emits light in a wavelength range that excites the second substance so it emits light that is detectable by photodetectors (8) and/or that is in the range of 600 nm to 900 nm.

4. Method of any of the preceding claims, wherein the luminophore substance comprises scintillation particles like LaF3:Tb3+ nanoparticles and/or LaF3:CE3+, Tb3+ nanoparticles.

5. Method of any of the preceding claims, wherein in step b) the ionizing radiation (4) is X-ray radiation or gamma radiation.

6. Method of any of the preceding claims, wherein in step b) the ionizing radiation (4) has a fan-like shape.

7. Method of any of the preceding claims, wherein in step b) the ionizing radiation (4) is irradiated in short pulses and/or by modulating the source (3) that emits the ionizing radiation (4) with a certain frequency.

8. Method of any of the preceding claims, wherein steps b) and c) are repeated and for each iteration the incidence angle of the ionizing radiation (4) to the object (3) is changed by moving the source (3) of the ionizing radiation along an orbital path (10) around the object (1).

9. Method of any of the preceding claims, wherein in step b) the object (1) is additionally irradiated by a non-ionizing light source.

10. Method of any of the preceding claims, wherein for the detection in step c) a plurality of photodetectors (8) is arranged on each side of the object (1).

11. Method of any of the preceding claims, wherein in step c) the whole emitted light power is detected and in step d) the detected light is analyzed by integrating over a closed surface around the target object (1).

12. Method of any of the preceding claims, wherein in step b) X-ray radiation is used and in step c) the X-ray radiation permeating the object (1) is detected by at least one X-ray detector (5).

13. Apparatus for diffuse luminescence probing in strongly scattering objects (1) according to the method of any of the claims 1 to 10, the apparatus comprising at least one source of ionizing radiation (3) and at least one photodetector (8) and a processing unit (9) for analyzing the data from the detector (8).

14. Apparatus of claim 13, further comprising at least one detector for ionizing radiation (5) for performing the method of any of the claims 1 to 12 and/or further comprising at least one non-ionizing light source.

15. Apparatus of claim 13 or 14, wherein the source of ionizing radiation (3) is movable along an orbital path (10) around the object (1).
